# EUROPEAN PATENT SPECIFICATION

(11) **EP 4 702 163 B1**
(45) Date of publication and mention of the grant of the patent: **14.10.2026**
(21) Application number: 24722607.9
(22) Date of filing: 28.04.2024
(51) Int. Cl.: C12Q 1/6886

(54) **METHODS FOR DIAGNOSING A HOMOLOGOUS RECOMBINATION DEFICIENCY IN HUMAN TUMORS**
VERFAHREN ZUR DIAGNOSE EINES MANGELS AN HOMOLOGER REKOMBINATION BEI MENSCHLICHEN TUMOREN
PROCEDES DE DIAGNOSTIC D'UNE DEFICIENCE DE RECOMBINAISON HOMOLOGUE DANS DES TUMEURS HUMAINES

(30) Priority: 28.04.2023 EP 23170829
(43) Date of publication of application: 04.03.2026
(73) Proprietor: Institut Curie, 75005 Paris (FR); Institut National de la Santé et de la Recherche Médicale, 75013 Paris (FR)
(72) Inventor: STERN, Marc-Henri, 75005 Paris (FR); POPOVA, Tatiana, 75005 Paris (FR); CALLENS, Céline, 75005 Paris (FR); EECKHOUTTE, Alexandre, 94800 Villejuif (FR)
(74) Representative: Plasseraud IP
(86) International application number: PCT/EP2024/061709
(87) International publication number: WO 2024/223927

(56) References cited:
- WO-A1-2021/177898
- WO-A1-2022/272310
- EECKHOUTTE ALEXANDRE ET AL: "ShallowHRD: detection of homologous recombination deficiency from shallow whole genome sequencing", vol. 36, no. 12, 21 April 2020 (2020-04-21), GB, pages 3888 - 3889, XP055857125, ISSN: 1367-4803, Retrieved from the Internet <URL:https://watermark.silverchair.com/btaa261.pdf?token=AQECAHi208BE49Ooan9kkhW_Ercy7Dm3ZL_9Cf3qfKAc485ysgAAAvswggL3BgkqhkiG9w0BBwagggLoMIIC5AIBADCCAt0GCSqGSIb3DQEHATAeBglghkgBZQMEAS4wEQQMYGaNR-PPEAWS1bs-AgEQgIICrrreWbbFygA0sgAM__BexquE37EZ1m7O6ndCFoHFFrv9TqYS6AgX0vXwmj1U_mGTxXCWeDK16QUJuky8Cq4uyYdVKcj_> DOI: 10.1093/bioinformatics/btaa261
- LIU YANG ET AL: "Homologous recombination deficiency prediction using low-pass whole genome sequencing in breast cancer", CANCER GENETICS, vol. 272-273, 1 April 2023 (2023-04-01), AMSTERDAM, NL, pages 35 - 40, XP093091488, ISSN: 2210-7762, DOI: 10.1016/j.cancergen.2023.02.001
- CAO WEN-MING: "Development of a new HRD algorithm which was highly associated with anthracyclinecontaining neoadjuvant chemotherapy", 1 June 2022 (2022-06-01), pages 1 - 1, XP093091490, Retrieved from the Internet <URL:https://ascopubs.org/doi/abs/10.1200/JCO.2022.40.16_suppl.e13575> [retrieved on 20231013]
- CALLENS C ET AL: "38MO Validation study of the ShallowHRDv2 assay for homologous recombination deficiency (HRD) detection in high-grade ovarian carcinomas (HGOC) in the first-line setting, from the phase III PAOLA-1/ENGOT-ov25 trial", vol. 8, no. 1, Supplement 1, 1 February 2023 (2023-02-01), pages 6 - 7, XP009548733, ISSN: 2059-7029, Retrieved from the Internet <URL:https://dul.usage.elsevier.com/doi/> DOI: 10.1016/J.ESMOOP.2023.100818
- DAGHER J ET AL: "Shallow whole genome sequencing accurately detects homologous recombination deficiency in ovarian cancer", vol. 481, no. SUPPL 1, Suppl. 1, Sp. Iss. SI, 31 August 2022 (2022-08-31), pages S59, XP009548732, ISSN: 0945-6317, Retrieved from the Internet <URL:http://www.springerlink.com/content/100455/>

## Description

### Technical Field

The present invention pertains to methods for diagnosing a homologous recombination deficiency in tumors.

### Background Art

*BRCA1*/*2*-mutated tumor cells are defective for the HR pathway (homologous recombination deficiency or HRD) and thus rely on alternative DNA repair pathways to avoid cell death, such as non-homologous end joining (NHEJ), alternative end joining (AltEJ), single strand annealing (SSA) or base excision repair (BER), all implicating PARP1 and PARP2 enzymes (Groelly et al., Nat Rev Cancer, 2022). As a consequence, inhibition of these PARP1/2 enzymes in *BRCA1*/*2*-deficient tumor cells results in cell death (Bryant et al., Nature 434:913-7, 2005; Farmer et al., Nature 434:917-21, 2005). PARPi represent a major progress in the treatment of *BRCA1*/*2*-deficient tumors. Several clinical trials have demonstrated that the maintenance with a PARP inhibitor with or without bevacizumab following platinum-based therapy improved progression-free survival in advanced ovarian cancer (AOC) patients with BRCA1/2-deficiency or HRD (Moore et al., N Engl J Med, 2018; Ray-Coquard et al., N Engl J Med 381:2416-2428, 2019; Gonzalez-Martin et al., N Engl J Med 381:2391-2402, 2019; Coleman et al., Lancet 390:1949-1961, 2017). As in the PAOLA-1 trial, a significant PFS benefit was observed in patients with HRD-positive tumors, including those without a *BRCA1*/*2* mutation (BRCAmut), according to the Myriad MyChoice CDx Plus test (MG test). The olaparib (ova)+bevacizumab (bev) maintenance regimen was thus approved in USA/Europe/Japan for patients with BRCAmut or HRD positive tumors. However, the MG test gave a substantial part of non-contributive results and was centralized until very recently.

Therefore, new reliable and feasible decentralized HRD tests need to be developed.

The European HRD ENGOT initiative (EHEI) is a unique collaboration of European academic laboratories with the aim of providing a reliable biomarker of HRD for selecting AOC patients who could benefit most from PARPi ± bevacizumab in first-line therapy (Pujade-Lauraine et al., International Journal of Gynecologic Cancer 31:A208-A208, 2021).

Recently, a new HRD test named shallowHRD, based on shallow / low coverage Whole Genome Sequencing (sWGS) showed good performance on fresh frozen samples (Eeckhoutte et al: Bioinformatics 36:3888-3889, 2020). sWGS is an easy and cheap technique, which can be applied to clinical samples, including formalin-fixed paraffin embedded (FFPE) samples. The bioinformatics pipeline of shallowHRD is rather straightforward and computationally light. However, poor performance in noisy samples (frequent for clinical FFPE) and significant number of non-resolved cases around HRD cut-off hampered the clinical application of this test.

Therefore, although being extremely promising, the shallowHRD needs significant improvements to meet the specificity and selectivity required for qualifying as an efficient test for the detection of HRD in samples such as FFPE samples.

### Summary

The invention is defined by the claims.

The present inventors have managed to significantly improve the performances of the shallowHRD and have made it suitable for routine clinical application.

The present inventors have particularly upgraded the shallowHRD in two basic aspects, (1) detection of LGA number, which became more reliable even in low quality samples; and (2) decision rules for diagnostics, which became more detailed, thereby providing diagnostics for patients who were classified as having an intermediate LGA number, i.e. who could not be directly classified as presenting a HRD or a homologous recombination proficiency (HRP).

The present inventors have added specific steps to the shallowHRD, which, as explained above consists in evaluating the number of large-scale genomic alterations (LGA) from a copy number alteration (CNA) profile obtained by shallow coverage whole genome sequencing (sWGS), so as to improve its precision. According to the shallowHRD, LGA number is calculated directly from CNA profile and the decision rule is based on the number of LGA with a large uncertainty area around the cut-offs (borderline). The major improvements brought by the present inventors consist in more precise LGA detection (due to noise reduction and quality control) and reliable diagnostics for cases with the borderline LGA number (due to additional decision rules). These added steps particularly comprise noise reduction, a quality classification of the CNA profile and selection of adaptive thresholds for LGA call, determining specific genomic markers, such as genome complexity, amplifications, etc, and multi-steps diagnostic procedure.

Therefore, according to a first embodiment, the present invention pertains to a method for diagnosing a homologous recombination deficiency in a tumor, said method comprising the steps of:
- evaluating the number of large-scale genomic alterations by obtaining the copy number alterations profile by shallow coverage whole genome sequencing (sWGS) in a tumor sample,
- determining a LGA-score (also referred to as "HRD score") which corresponds to the LGA number adjusted by the genome complexity of the tumor and the presence of a marker selected from the group of markers consisting of (1) cyclin dependent kinase 12 (CDK12) mutation-associated phenotype with multiple interstitial gains in the CNA profile, (2) Cyclin E1 (CCNE1) amplification, (3) human epidermal growth factor receptor-2 (HER2) amplification and (4) multifocal amplification phenotype.

The method according to the present invention is simple, easily reproducible and allows for the correct diagnosis of HRD in compromised-quality samples such as paraffin-embedded samples. The present invention therefore allows for the identification of patients who will benefit from a therapy comprising PARP inhibitors and/or an alkylating agents.

Therefore, according to another embodiment, the present invention pertains to a PARP inhibitor (PARPi) and/or an alkylating agent for use in a method for treating a cancer in a patient, wherein said patient has been diagnosed as having a tumor presenting a HRD according to the method of the present invention.

The present invention also pertains to a method for predicting the efficacy of a treatment in a patient suffering from cancer, wherein said treatment comprises a PARPi and/or an alkylating agent, and wherein said method comprises diagnosing HRD in a tumor sample as described herein.

### Brief Description of Drawings

**Figure 1****:** Shallow WGS approach and the main steps of shallowHRDv2 pipeline for effective HRD diagnostics. Abbreviations: CNA for Copy number alteration; FF for Fresh frozen; FFPE for formaldehyde fixed and Paraffin embedded; HRD for Homologous Recombination Defect.
**Figure 2****:** Conceptual workflow of shallowHRDv2 pipeline.
**Figure 3****:** Kaplan-Meier estimates of (A) PFS for PAOLA-1 patients according to homologous recombination status as determined with shallowHRDv2 or MyChoice and to the treatment arm. (B). OS for PAOLA-1 patients according to homologous recombination status as determined with shallowHRDv2 or MyChoice and to the treatment arm. (C) PFS according to homologous recombination status as determined with shallowHRDv2 and to the treatment arm for PAOLA-1 patients with non-contributive results by MyChoice. HR for Hazard Ratio; PFS for Progression Free Survival; OS for Overall Survival.
**Figure 4****:** Kaplan-Meier estimates of (A) PFS; (B) OS according to homologous recombination status as determined with shallowHRDv2 or MyChoice and to the treatment arm for PAOLA-1 patients with tumors wild-type for *BRCA1*/*2* genes. HR for Hazard Ratio; PFS for Progression Free Survival; OS for Overall Survival.
**Figure 5****:** Two examples (A and B) of training set and quality attribution used for modulation of the decision rules. In A, the numbers refer to the number of cases in the training set with corresponding tumor content and noise characteristics.
**Figure 6****:** Example of LGA SCORE distribution in the training data set (A) and adaptive model of SCORE modification for "borderline" SCOREs (B).
**Figure 7****:** Another example of LGA-score distribution in the training data set (A), adaptive model of LGA-score modification for "borderline" cases (B) and basic features of HRD and nonHRD cases with the Borderline LGA-scores.

### Detailed description

The present invention pertains to an improved method for the diagnosis of homologous recombination deficiency (HRD) in a tumor. The method according to the invention aims at adjusting the diagnosis obtained by evaluating the number of large-scale genomic alterations (LGA) by shallow coverage whole genome sequencing (sWGS).

According to a first aspect, the present invention pertains to a method for diagnosing a homologous recombination deficiency in a tumor, said method comprising the steps of:
- evaluating the number of large-scale genomic alterations by obtaining the copy number alterations profile by shallow coverage whole genome sequencing (sWGS) in a tumor sample,
- determining a LGA-score which corresponds to the LGA number adjusted by the genome complexity of the tumor and the presence of a marker selected from the group of markers consisting of (1) cyclin dependent kinase 12 (CDK12) mutation-associated phenotype with multiple interstitial gains in the CNA profile, (2) Cyclin E1 (CCNE1) amplification, (3) human epidermal growth factor receptor-2 (HER2) amplification and (4) multifocal amplification phenotype.

As used herein, the expression **"homologous recombination (HR) pathway"** has its general meaning in the art. It refers to the cellular pathway through which Double Stranded DNA breaks (DSB) are repaired by a mechanism called Homologous Recombination. Inside mammalian cells, DNA is continuously exposed to damage arising from exogenous sources such as ionizing radiation or endogenous sources such as byproducts of cell replication. All organisms have evolved different strategies to cope with these lesions. One of the most deleterious forms of DNA damage is DSB. HR is the most accurate mechanism to repair DSB because it uses an intact copy of the DNA from the sister chromatid or the homologous chromosome as a matrix to repair the break.

Cells (*e*.*g*., cancer cells) identified as having genomic DNA rearrangements (*e*.*g*., large-scale genomic alterations or LGA) can be classified as having an increased likelihood of having an HR deficiency, i.e. of having a deficient status in one or more genes in the HR pathway. As used herein, **"deficient status"** for a gene means the sequence, structure, expression and/or activity of the gene or its product is/are deficient as compared to normal. Examples include, but are not limited to, low or no mRNA or protein expression, deleterious mutations, hypermethylation, attenuated activity (*e*.*g*., enzymatic activity, ability to bind to another biomolecule), etc. As used herein, deficient status for a pathway (*e*.*g*., HR pathway) means at least one gene in that pathway (*e*.*g*., BRCA1) has a deficient status. Examples of highly deleterious mutations include frameshift mutations, stop codon mutations, and mutations that lead to altered RNA splicing. Deficient status in a gene in the HR pathway may result in deficient or reduced HR activity in cells (*e*.*g*., cancer cells).

Examples of genes in the HR pathway include, without limitation, BRCA1, BRCA2, PALB2/FANCN, BRIP1/FANCJ, BARD1, RAD51 and RAD51 paralogs (RAD51 B, RAD51C, RAD51D, XRCC2, XRCC3). These genes encode proteins that are important for the repair of double-strand DNA breaks by the HR pathway. When the gene for any such protein is, *e.g*., mutated or under-expressed, the change can lead to errors in DNA repair that can eventually cause cancer. Other actors of the HR pathway include FANCA, FANCB, FANCC, FANCD2, FANCE, FANCG, FANCI, FANCL, FANCM, FAN1, SLX4/FANCP or ERCC1.

Thus, the expression **"HR pathway deficiency"** or **"HRD",** as used herein, refers to a condition in which one or more of the proteins involved in the HR pathway for repairing DNA is deficient or inactivated. In contrast **"HR pathway proficiency"** or **"HRP"** refers to the absence of an HRD.

Proteins involved in the HR pathway can encompass, but are not limited to, inactivation of at least one of the following genes: BRCA1, BRCA2, PALP2/FANCN, BRIP1/FANCJ, BARD1, RAD51, RAD51 paralogs (RAD51B, RAD51C, RAD51D, XRCC2, XRCC3), FANCA, FANCB, FANCC, FANCD2, FANCE, FANCG, FANCI, FANCL, FANCM, FAN1, SLX4/FANCP and ERCC1.

As used herein the term **"inactivation",** when referring to a gene, can mean any type of deficiency of said gene. It includes but is not limited to germline mutations in the coding sequence, somatic mutations in the coding sequence, mutations in the promoter and methylation of the promoter.

The method according to the present invention allows diagnosing HRD in a tumor.

According to the present invention, the **"tumor"** can be any solid tumor or carcinoma. Preferably, the solid tumor or carcinoma is selected from breast cancer, ovary cancer, colon cancer, lung cancer, prostate cancer, renal cancer, metastatic or invasive malignant melanoma, brain tumor, bladder cancer, fallopian tube cancer, head and neck cancer, peritoneal cancer, liver cancer, bladder, breast, colon, kidney, liver, lung, pancreas, stomach, oesophagus, uterine, cervix, thyroid or skin cancer, including squamous cell carcinoma. However, the present invention also contemplates hematopoietic tumors such as leukemia, acute lymphocytic leukemia, acute lymphoblastic leukemia, B-cell lymphoma, T-cell lymphoma, Hodgkin's lymphoma, non-Hodgkin's lymphoma, hairy cell lymphoma, Burkitt's lymphoma, acute and chronic myelogenous leukemias and promyelocytic leukemia.

According to a specific embodiment, the tumor is selected from ovarian cancer, breast cancer, fallopian tube cancer, peritoneal cancer, lung cancer, pancreatic cancer, head and neck cancer, prostate cancer stomach or esophagus cancer, uterine cancer, cervical cancer, kidney cancer and bladder cancer.

According to a particular embodiment, the tumor is a breast cancer or an ovarian cancer, such as advanced ovarian cancer.

The tumor **"sample"** used in the context of the present invention is typically obtained from a tumor biopsy. It can e.g. be a fresh or a preserved sample such as a frozen sample, or any tumor sample preserved by other means. The tumor sample can typically be in the form of a formalin-fixed paraffin-embedded (FFPE) sample. An FFPE sample consists in a tumor sample fixed in formaldehyde, and then embedded in a paraffin wax block. FFPE samples are routinely prepared and used by the skilled person.

As used herein, the term **"patient"** or **"subject"** denotes a mammal, such as a rodent, a feline, a canine, a bovine, an equine, a sheep, a porcine or a primate. Preferably, a patient according to the invention is a human.

**"Large-scale genomic alterations"** or **"LGA"** correspond to a genomic rearrangement. LGA refers to any somatic copy number transition (e.g., breakpoint) along the length of a chromosome where is between two regions of at least some minimum length (e.g., at least 3, 4, 5, 6, 7, 8, 9, 10, 11 12, 13, 14, 15, 16, 17, 18, 19 or 20 or more megabases) after filtering out regions shorter than some maximum length (*e.g.,* 0.1, 0.2, 0.3, 0.4, 0.5, 0.6, 0.7, 0.8, 0.9, 1, 1.5, 2, 2.5, 3, 3.5, 4 or more megabases). For example, if after filtering out regions shorter than 3 megabases the somatic cell has a copy number of 1:1 for, *e*.*g*., at least 10 megabases and then a breakpoint transition to a region of, *e*.*g*., at least 10 megabases with copy number 2:2, this is an LGA. An alternative way of defining the same phenomenon is as an LGA Region, which is genomic region with stable copy number across at least some minimum length (*e*.*g*., at least 3, 4, 5, 6, 7, 8, 9, 10, 11 12, 13, 14, 15, 16, 17, 18, 19 or 20 megabases) bounded by breakpoints (*e*.*g*., transitions) where the copy number changes for another region also at least this minimum length. For example, if after filtering out regions shorter than 3 megabases the somatic cell has a region of at least 10 megabases with copy number of 1:1 bounded on one side by a breakpoint transition to a region of, *e*.*g*., at least 10 megabases with copy number 2:2, and bounded on the other side by a breakpoint transition to a region of, e.g., at least 10 megabases with copy number 1:2, then this is two LGAs. Notice that this is broader than allelic imbalance because such a copy number change would not be considered allelic imbalance (because the copy proportions 1:1 and 2:2 are the same, e.g., there has been no change in copy proportion). LGA and its use in determining HRD is described in detail in Popova et al., (Ploidy and large-scale genomic instability consistently identify basal-like breast carcinomas with BRCA1/2 inactivation, CANCER RES. (2012) 72:5454-5462).

According to the present invention, the number of LGA is determined by shallow coverage whole genome sequencing. **"Shallow coverage whole genome sequencing"** or **"sWGS"** allows detecting the number of LGAs obtained from whole genome sequencing (WGS) at low coverage, e.g. at a coverage from 0.3 and above. E.g. at a ~1X coverage. The coverage corresponds to the average number of reads that align to known reference bases. In the context of the present invention, sWGS is used so as to provide a **"copy number alteration profile"** or **"CNA"** profile. The method allowing for the determination of said profile based on sWGS is fully disclosed in Eeckhoutte et al (Bioinformatics 36:3888-3889, 2020). This method, referred to as **"ShallowHRD",** consists in processing sWGS of tumor samples with the Control-FREEC software (Boeva et al. (2012), Bioinformatics, 28, 423-425). This tool takes as input 'sample_name.bam_ratio.txt', which includes CNA profile {x, g}_{1, N} where x is normalized read counts in a sliding window, g is genomic coordinate and the profile segmentation with Sᵢ, Zᵢ segment median and size (in megabases, Mb).

ShallowHRD provides a CNA profile based on the following workflow:
1. CNA cut-off is detected and the profile segmentation is optimized as follows: Segments are defined as 'large' if Zᵢ ≥ (Q₁+Q₃)/2, where Q₁, Q₃ are quartiles of Zᵢ (Zᵢ > 3 Mb) distribution. M is detected as the first local minimum of |(Sᵢ-Sⱼ)| density, where i, j are large segments. CNA cut-off= min(max(0.025, *M*)*,* 0.45). Adjacent segments are merged if |(Sᵢ-Sᵢ₊₁)| < CNA cut-off; starting from the largest segment.
2. LGAs defined as intra-chromosome arm CNA breaks with adjacent segments Zᵢ,Zᵢ₊₁≥10Mb, are counted after removing segments <3 Mb.
3. The sample is annotated as 'non-HRD' (LGA < 15), 'borderline' (15 ≤ LGA ≤ 19) or 'HRD' (LGA > 19).
4. The sample quality is defined by *M* and *cMAD,* cMAD =median(|(x-Sₓ)|), where Sₓ corresponds to the segment enclosing x, before optimization: 'bad' (*cMAD >* 0.5 | *cMAD >* 0.14 and *M* > 0.45), 'average' (*cMAD >* 0.14 and M < 0.45 | *cMAD <* 0.14 and *M* > 0.45) or 'normal or highly contaminated' (*M* < 0.025).
*5. CCNE1* amplification is called if S_{c} ≥ 4·CNA cut-off, where *c* is the segment enclosing the gene.

In the context of the present invention, the diagnosis is provided based on a LGA-score, also referred to as "HRD score" which corresponds to the LGA number adjusted by the genome complexity of the tumor and the presence of a (i.e. 1, 2, 3 or all 4) marker selected from the group of markers consisting of (1) cyclin dependent kinase 12 (CDK12) mutation-associated phenotype with multiple interstitial gains in the CNA profile, (2) Cyclin E1 (CCNE1) amplification, (3) human epidermal growth factor receptor-2 (HER2) amplification and (4) multifocal amplification phenotype.

In the context of the present invention, the score is correlated with HRD: high LGA-scores are associated with HRD and low LGA-scores are associated with HRP. For example, according to the specific embodiment illustrated in the present examples, high scores would correspond to a score of 20, or alternatively 23 and above and low scores would correspond to a score of 17 and below.

Estimation of the genome complexity is performed by assigning the samples to two categories: "simple" and "complex", where "simple" genome has two most abundant copy number levels accounting for more than 70% of the genome. Otherwise, the genome is classified as "complex". According to a further embodiment, the genome can also be classified as "complex+", which is a subtype of "complex" that accounts for more than three equally abundant copy number levels; all low tumor content cases are annotated as "simple".

In the context of the present invention, a "simple" genome is considered to be correlated with HRD. Accordingly, a simple genome is assigned a modifying constant, that can be qualified as "positive" or as a "bonus", which will adjust the diagnosis towards HRD.

In contrast, a "complex" genome is considered to be correlated with HRP. Accordingly, a "complex" genome is assigned a modifying constant, that can be qualified as "negative" or as a "penalty", which will adjust the diagnosis towards HRP.

The method according to the present invention comprises adjusting the LGA number based on the presence of 1, 2, 3 or 4 marker(s) selected from the group of markers consisting of (1) cyclin dependent kinase 12 (CDK12) mutation-associated phenotype with multiple interstitial gains in the CNA profile, (2) Cyclin E1 (CCNE1) amplification, (3) human epidermal growth factor receptor-2 (HER2) amplification and (4) multifocal amplification phenotype.

**"Cyclin dependent kinase 12"** or **"CDK12"** is a protein kinase that acts as a key regulator of transcription elongation. It regulates the expression of genes involved in DNA repair and is required for the maintenance of genomic stability. The amino acid sequence of human CDK12 is available under reference Q9NYV4 in the Uniprot database. CDK12 is encoded by the *CDK12* gene which has the nucleic acid sequence as shown under reference ENSG00000167258 in the Ensembl genome database.

According to the present invention the LGA number is adjusted based on the presence of **"CDK12 mutation-associated phenotype with multiple interstitial gains in the CNA profile".** This marker refers to the detection of *CDK12* mutation-associated (CDK12mut) tandem duplication phenotype based on the number of interstitial gains of 1-10Mb (Popova et al, 2016). In the context of the present invention, CDK12mut-associated is detected based on the number of interstitial gains of 1-10Mb prevalent as compared with smaller interstitial gains and/or interstitial losses.

In the context of the present invention, the presence of a CDK12 mutation-associated phenotype with multiple interstitial gains in the CNA profile is considered to be correlated with HRP. Accordingly, the presence of this marker is assigned a modifying constant, that can be qualified as "negative" or as a "penalty" on a scale ranging from HRP to HRD, and will therefore adjust the diagnosis towards HRP.

**"Cyclin E1"** or **"CCNE1"** is a regulatory protein that is essential for the control of the cell cycle via its interaction with CDK2. The amino acid sequence of the CCNE1 protein is available under reference P24864 in the Uniprot database, and the sequence of the human *CCNE1* gene is shown under reference ENSG00000105173 in the Ensembl genome database.

According to the present invention the LGA number is adjusted based on **"CCNE1 amplification".** This marker is defined as the fact that chromosome 19 carries high copy number gains (e.g. amplification) of the *CCNE1* locus.

In the context of the present invention, CCNE1 amplification is considered to be correlated with the presence of HRP. Accordingly, the presence of a CCNE1 amplification is assigned a modifying constant, that can be qualified as "negative" or as a "penalty", which will adjust the diagnosis towards HRP.

**"Human epidermal growth factor receptor-2"** or **"HER2",** also referred to as **"receptor tyrosine-protein kinase erbB-2", "ERBB2", "cluster of differentiation 340"** or **"CD340"** is a protein tyrosine kinase encoded by the *ERBB2* gene. The amino acid sequence of the HER2 protein is available under reference P04626 in the Uniprot database, and the sequence of the human *ERBB2* gene is shown under reference ENSG00000141736 in the Ensembl genome database.

According to the present invention the LGA number is adjusted based on the detection of **"HER2 amplification".** This marker is defined as the fact that chromosome 17 carries high copy number gains (e.g. amplification) of the *HER2* locus.

In the context of the present invention, HER2 amplification is considered to be correlated with the presence of HRP. Accordingly, the presence of a HER2 amplification is assigned a modifying constant, that can be qualified as "negative" or as a "penalty", which will adjust the diagnosis towards HRP.

According to the present invention the LGA number is adjusted based on the detection of **"multifocal amplification phenotype".** This marker is defined as the fact that more than two chromosome arms carry at least one high copy gain (amplification) of any given region.

In the context of the present invention, **multifocal amplification** is considered to be correlated with the presence of HRP. Accordingly, the presence of a multifocal amplification is assigned a modifying constant, that can be qualified as "negative" or as a "penalty", which will adjust the diagnosis towards HRP.

When the tumor sample is a FFPE sample, the method according to the present invention can advantageously comprise a step wherein the CNA profile from which said LGA number is calculated, is corrected by eliminating, i.e. significantly reducing, the false positive breakpoints that correlate with the noise profile, i.e. by determining the FFPE cumulative noise profile. Noise correction is advantageously associated with a segmentation optimization.

This step is advantageously performed prior to determining the HRD score as explained above.

**FFPE cumulative noise profile** is obtained from segmented CNA profiles of ~100 almost normal genomes sequenced from FFPE tumor or normal samples. Each segment average is replaced by 1 or -1, if upper or lower than general average value, respectively. Each genomic bin is thus characterized by the sum of 1/0/-1 from ~100 profiles. When the sample is a FFPE tumor sample, the FFPE noise profile has clear peaks and holes at certain positions in the genomes. Correlation > 0.2 between tumor CNA and FFPE cumulative profiles and proportion of the genome falling in large segments (>20Mb) after segmentation < 40% are characteristic of high FFPE noise.

**Segmentation optimization** is performed by filtering small segments and merging the segments with small differences in average value (small differences means less than a threshold). FFPE noise reduction is performed under the same logic, adjacent segments are merged together if their dynamic is correlated locally to the FFPE cumulative noise profile.

A threshold for between segment difference to be considered as negligible is selected depending on the noise and tumor content estimation. After all adjacent segments with small difference in the average values are merged, the profile is considered to be optimized. Thresholds for CNA, i.e. breakpoint calls, are fitted for each sample quality category and usually set to be twice as large.

As mentioned above, the method according to the present invention can advantageously be used for resolving border cases, i.e. cases for which the score does not correspond to HRD and does not correspond to HRP either. For instance, when the score is calculated as shown in the Examples below, borderline cases correspond to a LGA-score of 17< LGA-score < 20 or alternatively 23. In such cases, the method according to the present invention further comprises resolving, i.e. further adjusting, the score by taking into account:
the genome complexity of the tumor;
the presence of a marker selected from the group of markers consisting of (1) cyclin dependent kinase 12 (CDK12) mutation-associated phenotype with multiple interstitial gains in the CNA profile, (2) Cyclin E1 (CCNE1) amplification, (3) human epidermal growth factor receptor-2 (HER2) amplification and (4) multifocal amplification phenotype; and
an ancillary cumulative LGA index (LGA_boost) or alternatively a LGA_max corresponding to the upper bound of LGA counts found in the optimized segmented genomic profile not restricted by the thresholds for LGA call).

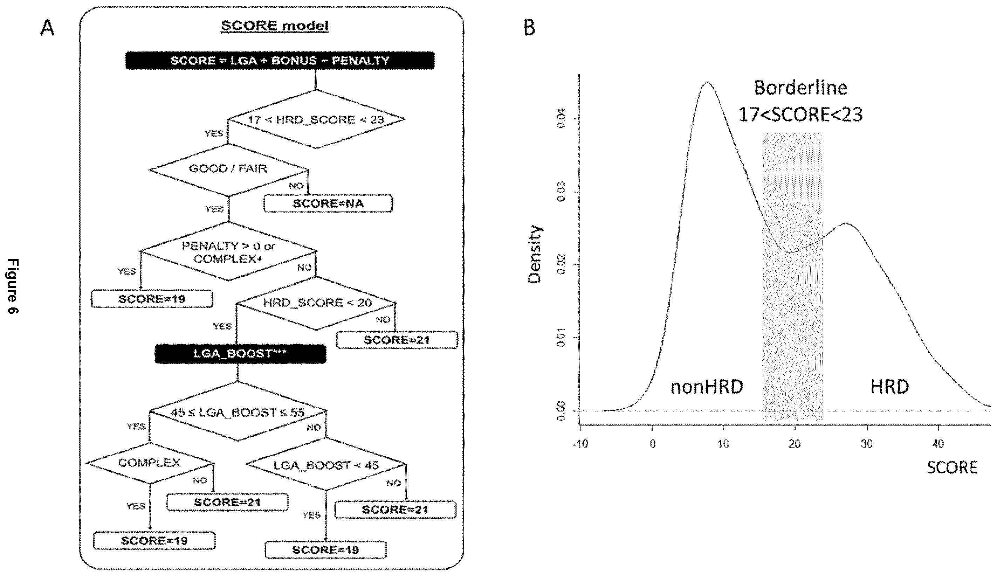

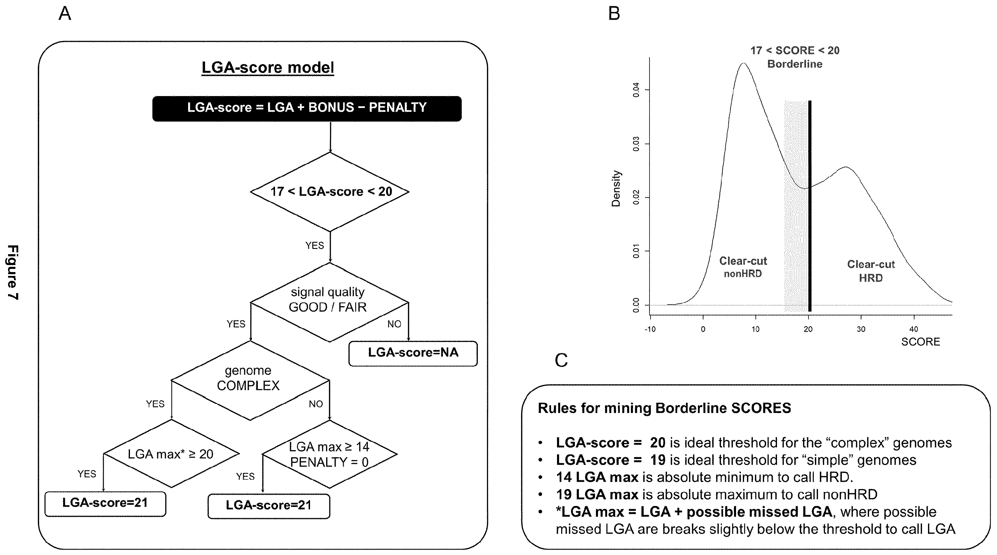

Genome complexity and the presence of a marker selected from the group of markers consisting of (1) cyclin dependent kinase 12 (CDK12) mutation-associated phenotype with multiple interstitial gains in the CNA profile, (2) Cyclin E1 (CCNE1) amplification, (3) human epidermal growth factor receptor-2 (HER2) amplification and (4) multifocal amplification phenotype are determined as explained above. The method for further adjusting the score for borderline cases based on these elements is disclosed in Figure 6 or Figure 7..

When adjusting the diagnosis with the ancillary cumulative LGA index (LGA_boost), said index is calculated by the following formula: 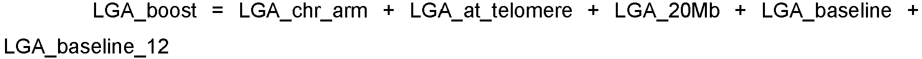 LGA_boost = LGA_chr_arm + LGA_at_telomere + LGA_20Mb + LGA_baseline + LGA_baseline_12 wherein
LGA_chr_arm is the number of chromosome arms with LGA;
LGA_at_telomere is the number of chromosome arms with LGA at telomeric end;
LGA_20Mb is the number of LGA with both genomic segments at copy number (CN) break being more than 19Mb;
LGA_baseline is the number of LGA involving the most abundant CN layer;
LGA_baseline_12 is the number of LGA detected between two most abundant CN layers.

The way LGA_boost is used for adjusting the diagnosis is explained in Figure 6.

When adjusting the diagnosis with the LGA_max index, said index is determined as being the upper bound of LGA counts found in the optimized segmented genomic profile not restricted by the thresholds for LGA call. LGA_max = LGA + possible missed LGA, where possible missed LGA have breakpoint amplitude less than a threshold for LGA call.

The way LGA_max is used for adjusting the diagnosis is explained in detail in the experimental section of the present application as well as in Figure 7.

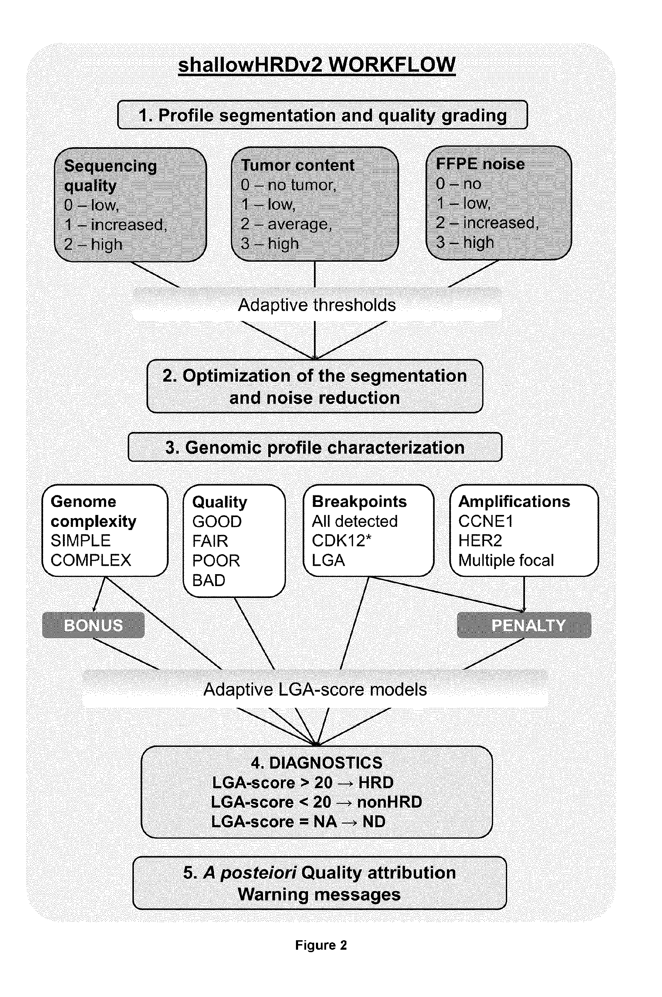

According to a preferred embodiment, the method according to the present invention comprises the following steps (Figure 2):
1) Obtaining a copy number alteration profile determined by evaluating the number of LGAs by sWGS in a tumor sample (by normalization of sWGS read counts profile in a tumor sample);
2) Noise reduction and segmentation optimization;
3) Genomic profile characterization:
   - Estimation of the genome complexity.
   - Overall signal quality attribution (four categories: "good", "fair", poor and "bad"), which is based on tumor content and noise, and defines the path to final diagnosis, including conditions for not determined status (ND).
   - CNA breakpoints analysis: call of large-scale genomic alterations (LGA), detection of *CDK12* mutation-associated (CDK12mut) tandem duplication phenotype, (Popova et al, Cancer Res 2016; PMID 26787835) based on the number of interstitial gains of 1-10Mb;
   - Check for *CCNE1* amplification, *ERBB2* amplification and multifocal amplification phenotype.
4) LGA-score and HRD status attribution and final diagnosis (i.e. HRD or HRP):
   LGA_SCORE = LGA + BONUS - PENALTY
   Rules for final diagnosis are based on the LGA-score, sample quality attribution and CNA profile characteristics.

Because it is possible to predict whether a given patient suffers from a cancer which is associated with HRD, it is also possible to select the appropriate therapy for said patient.

As described herein, patients having cancer cells identified as having a genomic DNA rearrangement (*e*.*g*., LGAs) can be classified as being likely to respond to a particular cancer treatment regimen. For example, patients having cancer cells with a genome containing a genomic DNA rearrangement can be classified, as being likely to respond to a cancer treatment regimen that includes the use of a DNA damaging agent, a synthetic lethality agent (*e*.*g*., a PARP inhibitor), radiation, or a combination thereof.

Therefore, another aspect of the present invention concerns a method for predicting the efficacy of a treatment in a patient suffering from cancer, wherein said treatment comprises a PARPi and/or an alkylating agent, and wherein said method comprises diagnosing HRD in a tumor sample as described above.

The invention also relates to a PARPi and/or an alkylating agent for use in a method for treating cancer in patient wherein said patient is diagnosed as having a tumor presenting a HRD according to the method of the present invention.

As used herein the term **"PARP inhibitor"** or **"PARPi"** has its general meaning in the art. It refers to a compound which is capable of inhibiting the activity of the enzyme polyADP ribose polymerase (PARP), a protein that is important for repairing single-strand breaks ('nicks' in the DNA). If such nicks persist unrepaired until DNA is replicated (which must precede cell division), then the replication itself will cause double strand breaks to form. Drugs that inhibit PARP cause multiple double strand breaks to form in this way, and in tumors with BRCA1, BRCA2 or PALB2 mutations these double strand breaks cannot be efficiently repaired, leading to the death of the cells.

Typically, the PARP inhibitor according to the invention can be selected from the group consisting of iniparib, olaparib, rocaparib, CEP 9722, MK 4827, BMN-673, and 3-aminobenzamide.

As used herein, the term **"alkylating agent"** or **"alkylating antineoplastic agent"** has its general meaning in the art. It refers to compounds which attach an alkyl group to DNA. Typically, the alkylating agent according to the invention can be selected from platinium complexes such as cisplatin, carboplatin and oxaliplatin, chlormethine, chlorambucil, melphalan, cyclophosphamide, ifosfamide, estramustine, carmustine, lomustine, fotemustine, streptozocin, busulfan, pipobroman, procarbazine, dacarabazine, thiotepa and temozolomide.

The method according to the present invention can advantageously be implemented by a computer, by using a computer program tailored for reproducing the steps disclosed above.

In another aspect, the invention provides a computer program product embodied in a computer readable medium that, when executed on a computer, provides instructions for evaluating the number of LGAs in a tumor sample, correcting the sample noise profile, providing an HRD diagnostic according to a method of the present invention.

### EXAMPLES

### Achieving clinical confidence in shallow whole genome sequencing approach for Homologous Recombination Deficiency detection in tumors

### METHODS:

### Patients and tumor samples

The first cohort consisted of FFPE-derived DNA from 449 AOC samples from the PAOLA-1/ENGOT-ov25 trial in the frame of the EHEI. All patients provided written informed consent. The second cohort consisted of 109 consecutive FFPE AOC samples (8 to 20 slides of 5µm according to the tumor area) which were sent to Myriad Genetics central laboratory (Salt Lake City, UT, USA), from March 2021 to January 2022, as part of the routine practice. In parallel, shallowHRDv2 was performed on the same FFPE samples in the Institut Curie genetics laboratory.

### Statistical analysis

The Kaplan-Meier method was used to estimate progression-free survival (PFS) and overall survival (OS), with the stratified log-rank test used to assess the difference between the Olaparib (ola)+bevacizumab (bev) group and the bev group. The hazard ratio (HR) and associated 95% confidence interval (95% CI) were calculated with the use of a stratified Cox proportional-hazards model. All statistical analyses were performed using GraphPad Prism software version 9.1.0.

### Shallow WGS workflow

100ng of FFPE DNA were used as input. Mechanical DNA shearing was performed using Covaris (model ME220 Focused-ultrasonicator) with 50 µl DNA samples. We followed supplier's recommendations for the Agilent kit (SureSelect XT HS and XT Low Input Library Preparation, G9703A). The different steps consisted of ligation, amplification and purification with AMpure XP beads (Beckman Coulter, ref: A63882). Dosages were performed with Thermo Fisher Scientific Qubit^{®} dsDNA HS Assay Kit (ref: Q32854) or Qubit^{®} dsDNA BR Assay Kit (ref: Q32853). After quality and quantity controls with Agilent TapeStation and D1000 ScreenTape, we prepared a library pool of 4nM or 1.8nM to the NextSeq 550 S or NovaSeq 6000 Sequencing systems, respectively (Illumina Inc, San Diego, CA, USA).

### ShallowHRDv2 bio-informatics pipeline

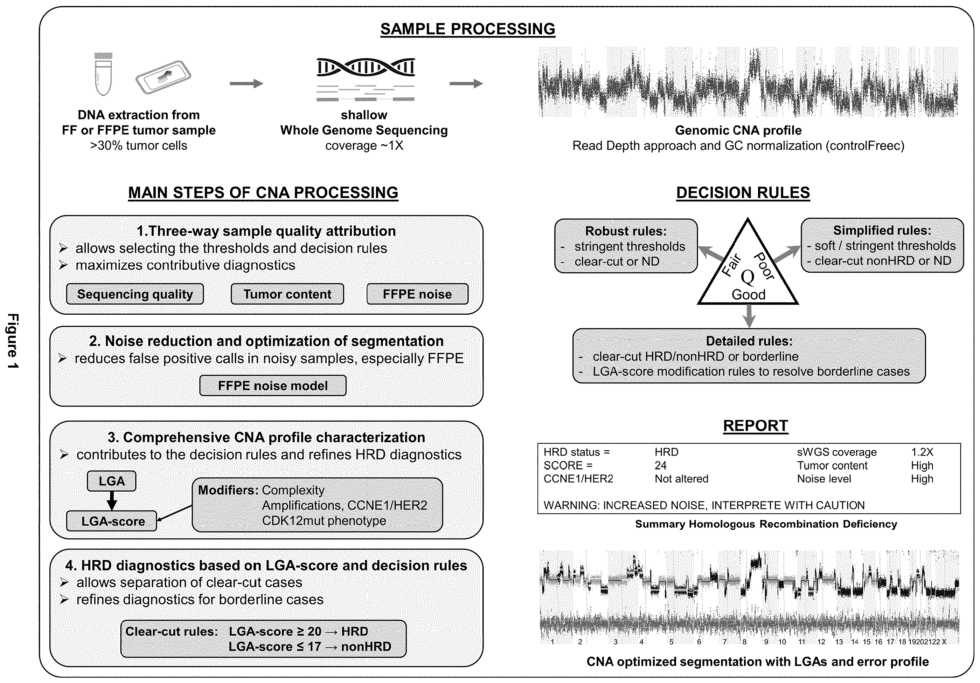

After DNA extraction and whole genome sequencing at low coverage (~1X), read counts profile (bin size ~50kb) normalized and corrected for GC-content was obtained by ControlFreec (Boeva, V., et al. Control-FREEC: a tool for assessing copy number and allelic content using next-generation sequencing data. Bioinformatics 28, 423-425 (2012)) (Figure 1). ShallowHRDv2 bioinformatics pipeline consists in the analysis of copy number alteration (CNA) profile providing HRD diagnostics, sample quality attribution and comprehensive quantitative and graphical output for manual control. Main steps of the pipeline, outline of the decision rules and diagnostics are described below and in Figure 1.

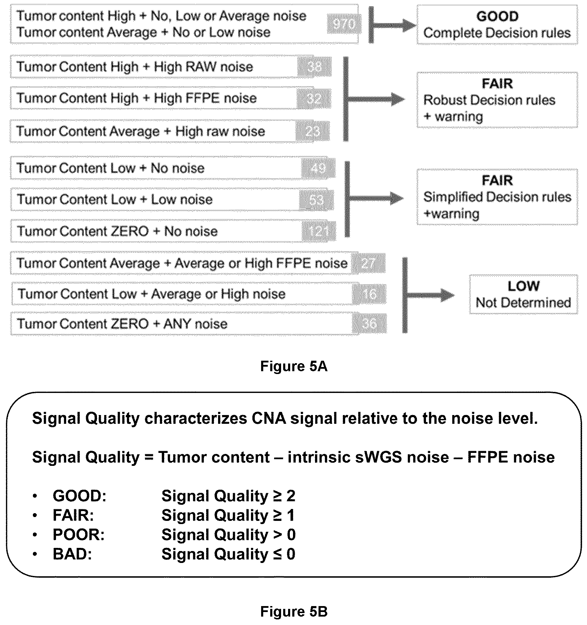

Main steps of CNA processing are (Figure 2):
(1) Three-way sample quality attribution: CNA profile classification according to tumor content (four categories), intrinsic sWGS noise (three categories) and FFPE noise (four categories) with final integrative classification of signal relative to noise in four categories: "good", "fair", "poor" and "bad" (Figure 5B).
(2) Noise reduction and optimization of breakpoints in the CNA profile: filtering small segments and assembling segments with small differences or local correlations to the FFPE noise profile (obtained from ~100 normal profiles from FFPE samples, Figure 1), with the thresholds for the breakpoint calls fitted for each quality category.
(3) Broad CNA profile characterization by:
   - genome complexity, where the "simple" genome has two most abundant copy number (CN) levels accounting for more than 70% of the genome, otherwise, the genome is classified as "complex";
   - a set of binary attributes, such as *CCNE1* amplification, *ERBB2* amplification, focal amplification phenotype (called when more than two chromosome arms carry at least one amplification), *CDK12* mutation-associated tandem duplication phenotype (called when multiple interstitial gains of 1-10Mb are detected) (Popova, T., et al. Ovarian Cancers Harboring Inactivating Mutations in CDK12 Display a Distinct Genomic Instability Pattern Characterized by Large Tandem Duplications. Cancer Res 76, 1882-1891 (2016));
   - a set of parameters characterizing the breakpoints, including overall number and the number of large genomic alterations (LGA), that largely contributes to the HRD diagnostics; LGA are defined as CN breaks between genomic segments of more than 9Mb (segment sizes are rounded to the integer number).
(4) Multi-step HRD diagnostics based on:
   - LGA-score, which is essentially the number of LGA modified by PENALTY and BONUS, where PENALTY is defined by binary attributes and is subtracted from the LGA number (PENALTY is set to 0, 5 or 8 if none, one or more than one binary attributes hold true, respectively) and BONUS is defined by the genome complexity and is added to the LGA number (BONUS is set to 5 for "simple" genome and to 0 otherwise);
   - two thresholds for clear-cut HRD diagnostics, namely 17 and 20, with the LGA-score < 17 for nonHRD and LGA-score ≥ 20 for HRD;
   - LGA-score modification, which is applied to resolve the borderline cases (17 < LGA_SCORE <20). Briefly, the LGA-score is shifted to 21 if evidence for HRD (genome is classified as "simple", PENALTY = 0 and LGA_max ≥ 14 or genome is classified as "complex" and LGA_max ≥ 20) (Figure 7).

*Decision rules.* Decision rules are multi-step, depend on sample quality attribution and include selection of the thresholds for LGA call. Two thresholds were utilized to call LGA at CNA breakpoint: stringent (implying simple genome) and soft (implying complex genome), which are applied in conservative manner for good quality cases (LGA-score was based on LGA number with soft/stringent thresholds for nonHRD/HRD clear-cut diagnostics) and are fixed to the stringent/soft ones for noisy/low tumor content samples, respectively. Simplified decision rules for bad quality samples consist in giving the diagnosis only for clear-cut nonHRD cases with small overall number of the breakpoints. Most of bad quality cases are discarded. Robust decision rules for poor quality samples consist in giving the diagnosis only for clear-cut cases, leaving the borderline cases with not determined (ND) diagnosis. Additional rules for LGA_score modification procedure for good/fair quality borderline cases help resolving the diagnostics and reduce non-determined cases.

*Comprehensive output (report).* Final diagnosis is reported along with quality assessment and warning messages. Quantitative output provides complete information on the decisive genomic biomarkers, LGA, LGA-score and HRD diagnostics. Output includes segmented profile with the LGAs detected and the error profile to visually control noise reduction quality and segmentation.

Circular binary segmentation of CNA profile has a stochastic component and can result in between-run variation in LGA number, which may affect the diagnostics if close to the thresholds. Thus, LGA n umber is reported as average estimated from 21 segmentation/optimization runs along with the standard error.

### Detailed workflow of shallowHRDv2.

**Step 1)** CNA profile segmentation using the circular binary segmentation method and CNA profile classification according to tumor content (four categories), intrinsic sWGS noise (three categories) and FFPE noise (four categories). Combination of these attributes provides overall sample quality attribute ("good", "fair", "poor" or "bad"), utilized in selection of the decisive pipeline and reporting (Figure 5B).
- Profile is characterized by the number of breakpoints; variance of CNA profile, within segment variance, between segments variance; correlation to FFPE noise; percent of the genome belonging to the segments >20Mb after segmentation.
- *Sequencing quality* is characterized by raw variance (variance within the segments).
- *Tumor content* is characterized by the variance of medians of the large segments.
- *FFPE noise* is characterized by the number of breakpoints, correlation to FFPE cumulative profile, variance of error profile and the proportion of large segments after initial profile segmentation. FFPE noise profile is obtained from segmented CNA profiles of ~100 almost normal genomes sequenced from FFPE samples. Each segment average is replaced by 1 or -1, if upper or lower than general average value, respectively. Each genomic bin is thus characterized by the sum of 1/0/-1 from ~100 profiles. Correlation > 0.2 between tumor CNA and FFPE cumulative profiles and proportion of the genome in the large segments (>20Mb) after segmentation < 40% are characteristic of high FFPE noise.

**Step 2)** Noise correction and segmentation optimization: filtering small segments and merging segments with small difference in median values or local correlations to the FFPE noise profile. A threshold for between segment difference to be considered as negligible (no breakpoint call) is selected depending on the noise and tumor content category. The adjacent segments were merged if the median difference is less than the threshold. The breakpoint(s) were also eliminated if it (they) followed the breakpoint in FFPE covariate profile even if the difference exceeded the threshold.

**Step 3)** Genomic profile characterization:
- Estimation of the genome complexity (two categories: "simple" and "complex", where "simple" genome has two most abundant copy number levels accounting for more than 70% of the genome. Otherwise, the genome is classified as "complex". All low tumor content cases are annotated as "simple".
- Overall profile quality attribution (four categories: "good", "fair", "poor" and "bad"), which is based on tumor content and noise, characterizes signal relative to noise and defines the path to final diagnosis, including conditions for not determined status (ND).
- CNA breakpoints analysis: LGA calling is performed after filtering out the segments less than 3Mb and merging adjacent large segments if between segment distance is less than 3Mb; LGA are called in adaptive mode, i.e. using two thresholds, stringent (implying simple genome) and soft (implying complex genome); stringent threshold is also applied in the noisy samples, while soft threshold is applied in low tumor content cases. "Possible missed LGA" count the LGA breakpoints with amplitude less than the soft threshold.

- detection of *CDK12* mutation-associated (CDK12mut) tandem duplication phenotype based on the number of interstitial gains of 1-10Mb {Popova et al, 2016};
- Check for *CCNE1* amplification, *ERBB2* (HER2) amplification and amplification phenotype (called when more than two chromosome arms carry at least one amplification).

**Step 4)** LGA-score and HRD status attribution (Figure 7A): $LGA_SCORE = LGA + BONUS - PENALTY ,$ where BONUS=5 for "simple" genome; PENALTY=5 if one amplification or CDK12mut phenotype is detected; PENALTY=8 if two or more of these features are detected.
- LGA-score distribution in the training set is shown in Figure 7B. The LGA-score less than 17 and the LGA-score more or equal than 20 are considered definitive (clear-cut); the LGA-score more than 17 and less than 20 is considered borderline.
- For borderline LGA-scores several modification rules are applied: the LGA-score is shifted to 21 if evidence for HRD (genome is classified as "simple", PENALTY = 0 and LGA_max ≥ 14 or genome is classified as "complex" and LGA_max ≥ 20) (Figure 7).
- Ancillary value LGA_max used to further clarify the HRD status of borderline cases corresponds to maximal number of LGAs in the tumor segmented profile (obtained if thresholds for LGA call are ignored).
- HRD is called if LGA-score ≥ 20 and nonHRD is called if LGA-score < 20. Borderline scores in POOR quality samples lead to ND diagnostics.
- Final diagnosis is reported along with quality assessment and warning messages.

Random initiation of segmentation algorithm and stochastic profile optimization by the system of fixed thresholds lead to possible variation in LGA number eventually affecting the final diagnosis. The complete workflow thus includes 11 runs to fix intermediate parameters and get preliminary diagnosis and error estimation for average LGA and LGA-score, followed by 10 runs to narrow the confidence intervals.

### RESULTS:

Institut Curie joined the EHEI initiative and had access to 449 DNA extracted from FFPE tumor samples from the PAOLA-1 trial to validate the shallowHRDv2 test. Characteristics of these 449 patients at baseline showed that they were representative of the global cohort. All samples were previously tested by MyChoice in the frame of the PAOLA-1 trial, allowing us to establish a concordance table with the binary classification resulting from GIS (HRD *versus* nonHRD) and shallowHRDv2. Of the 394 samples with a conclusive result from both tests (394/449; 88%), we observed an overall concordance of 94% (369/394), a positive agreement of 95% (196/206) and a negative agreement of 92% (173/188). The percentage of inconclusive tests were 11% (51/449) for GIS, while shallowHRDv2 yielded a failure rate of 3% (15/449). Cohen's Kappa at 0.73 (p=1.95x10⁻¹⁴⁸) indicated a substantial agreement between the two tests. The correlation between scores from MyChoice (GIS) and shallowHRDv2 (LGA) was good (R²=0.85), discordant cases concentrated around the thresholds of each test. Among the 15 GIS HRP/shallowHRDv2 HRD cases, six tumors carried a *BRCA1*/*2* pathogenic variant. Among 10 GIS HRD/shallowHRDv2 HRP cases, three tumors carried a *BRCA1*/*2* pathogenic variant. The information for association with a loss of heretozygosity was not available for B*RCA1*/*2* variants.

To assess the actual improvement of the v2 as compared with the v1 version of the shallowHRD pipeline, we evaluated the performance of shallowHRDv1 on the same samples of the PAOLA-1 trial. The performances of shallowHRDv1 in comparison with GIS were acceptable with an overall agreement of 93% (309/333), a positive agreement of 95% (169/177), a negative agreement of 90% (140/156) and 4% (17/449) of non-contributive results. Cohen's Kappa at 0.56 (p=8.25x10⁻⁷²) indicated a moderate agreement between the two tests, lower than that obtained with v2. Moreover, shallowHRDv1 calls a « borderline » status when the number of detected LGA is between 15 and 19 ("sensitive" and "specific" cut-offs). As much as 15% (66/449) of PAOLA-1 samples were called « borderline », according to shallowHRDv1, for which a diagnostic of HRD status would not have been delivered.

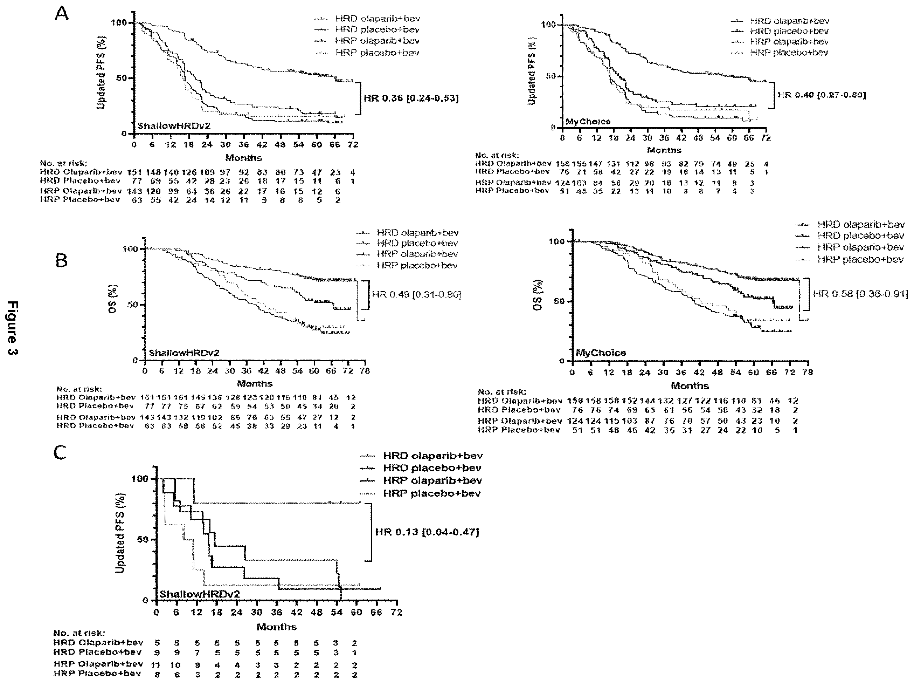

As shallowHRDv2 was highly correlated with MyChoice, we further evaluated if HRD according to shallowHRDv2 was predictive of the clinical benefit of ola+bev maintenance in this subset of the PAOLA-1 cohort (Ray-Coquard, I., et al. Olaparib plus Bevacizumab as First-Line Maintenance in Ovarian Cancer. N Engl J Med 381, 2416-2428 (2019); Ray-Coquard, I. L., et al. Final overall survival (OS) results from the phase III PAOLA-1/ENGOT-ov25 trial evaluating maintenance olaparib (ola) plus bevacizumab (bev) in patients (pts) with newly diagnosed advanced ovarian cancer (AOC). Ann Oncol 33, S808-S869 (2022)). The median duration of follow up was 63 months (interquartile range [IQR]: 28.5-62.3). According to shallowHRDv2, median PFS was 65.7 months in the HRD group (whatever the *BRCA1*/*2* status) treated with ola+bev and 20.3 months when treated with bev (HR 0.36 [95% Cl, 0.24-0.53]), whereas it was 57.1 months in the HRD ola+bev group and 20.1 months in the HRD bev group according to MyChoice (HR 0.40 [95% Cl, 0.27-0.60]; Figure 3A). ShallowHRDv2 also showed similar performance than MyChoice regarding OS results. According to shallowHRDv2 and MyChoice, median OS was 75.2 months in the HRD group treated with ola+bev and 66.4 months when treated with bev (HR 0.49 [95% Cl, 0.31-0.80] and HR 0.58 [95% Cl, 0.36-0.91] for shallowHRDv2 and MyChoice, respectively; Figure 3B).

Importantly, shallowHRDv2 was also predictive of the PARPi benefit for patients with a non-contributive MyChoice test. Median PFS in HRD patients according to shallowHRDv2 was not reached when treated with ola+bev as compared with a median PFS of 17.4 months when treated with bev alone (HR: 0.13 [95% Cl, 0.04-0.47]; Figure 3C).

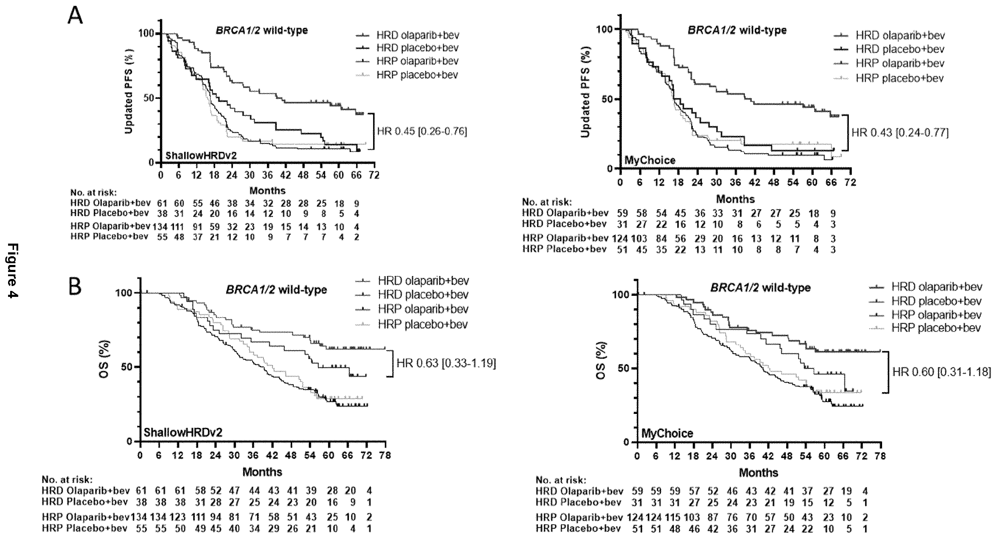

The main advantage of detecting HRD based on genomic profiles is to identify patients whose tumors might be deficient for homologous recombination in the absence of *BRCA1*/*2* pathogenic variants. Therefore, we evaluated the predictive value of shallowHRDv2 in this subgroup of patients with *BRCA1*/*2* wild-type (wt) AOC. Median PFS was 40.8 months with ola+bev and 19.5 months with bev (HR: 0.45 [95% Cl, 0.26-0.76]) in *BRCA1*/*2*wt, HRD tumors according to shallowHRDv2. According to MyChoice median PFS was 40.8 months with ola+bev and 17.6 months with bev (HR: 0.43 [95% Cl, 0.24-0.77]) in *BRCA1*/*2*wt, HRD tumors (Figure 4A). Of note, we noticed that across patients with BRCA1/2wt tumors receiving bev alone, those with HRD tumors according to shallowHRDv2 tended to have a longer PFS than patients with HRP suggesting a prognostic value of the HRD status but the difference was not statistically significant (p=0.28).

In HRD BRCA1/2wt tumors, the median OS was not reached in the ola+bev treated patients, whatever the test employed, *versus* a median OS in bev-treated patients of 56.6 and 55.0 months when HRD was defined by shallowHRDv2 or MyChoice, respectively (HR for comparison between ola+bev and bev: 0.63 [95% Cl, 0.33-1.19] and 0.60 [95% Cl, 0.31-1.18], defined by shallowHRDv2 or MyChoice, respectively; Figure 4B). In contrast, patients with HRP BRCA1/2wt tumors according to shallowHRDv2 receiving ola+bev tended to have a shorter median OS than those receiving placebo+bev although not significant (38.2 *versus* 42.1 months, respectively; log-rank p=0.55 Figure 3B).

ShallowHRDv2 showed good analytical performances and was equivalent to MyChoice to predict PARPi benefit in the PAOLA-1 cohort. However, these conclusions were done on samples issued from patients included in a clinical trial and might be different in routine diagnosis practice. Thus, we evaluated performances of shallowHRDv2 on an independent cohort of 109 unselected, consecutive, FFPE AOC cases issued from our routine laboratory, which were also submitted to MyChoice. We confirmed the high overall agreement of 91% (86/94) with 92% (36/39) positive agreement and 91% (50/55) negative agreement between shallowHRDv2 and MyChoice in this prospective cohort, with less non-contributive results for shallowHRDv2 (5% *versus* 12%). Cohen's Kappa at 0.69 (p=7.49x10⁻²⁹) confirmed a moderate agreement between the two tests

### DISCUSSION

Assessment of HRD status is mandatory to balance benefit and risks of PARPi maintenance in newly diagnosed OAC patients. Thus, there is a need for local, reliable, and cheap HRD tests. We report here the development of the shallowHRDv2 test, and its clinical validation showing its high concordance to MyChoice in the PAOLA-1 trial to predict the benefit of ola+bev in AOC patients. Main improvements of the v2 pipeline, as compared with the first version of the shallowHRD test (Eeckhoutte, A., et al. ShallowHRD: detection of homologous recombination deficiency from shallow whole genome sequencing. Bioinformatics 36, 3888-3889 (2020)) include FFPE noise correction, critical diagnostic assessment based on tumor content and sWGS noise level, improved binary HRD classification with a reduced number of non-resolved cases, and ancillary genomic features to refine the final conclusion. Thanks to these improvements, shallowHRDv2 reduces the number of non-conclusive results by ~60 to 75% as compared with MyChoice (3% *versus* 11% in the PAOLA-1 cohort and 5% *versus* 12% in the routine cohort). More importantly, these patients with shallowHRDv2 HRD status but non-contributive in MyChoice significantly benefit from ola+bev combination treatment, allowing more patients with AOC to benefit from ola+bev. With only 3.3% of non-contributive analyses, shallowHRDv2 robustness is similar to other genetic testing in clinical routine, such as *BRCA1*/*2* tumor testing, which showed a 4.4% failure rate in the PAOLA-1 clinical trial (Callens, C., et al. Concordance Between Tumor and Germline BRCA Status in High-Grade Ovarian Carcinoma Patients in the Phase III PAOLA-1/ENGOT-ov25 Trial. J Natl Cancer Inst 113, 917-923 (2021)).

We noticed that six cases carrying *BRCA1*/*2* pathogenic variants were misclassified HRP by MyChoice, while correctly classified HRD with shallowHRDv2.

The clinical value of HRD testing may not be restricted to the question of ola in AOC. Contrary to ola, which has not been authorized in first line for HRP cases (alone or in combination with bev), niraparib has obtained an "all-comers" approval.

Using a cheap and robust HRD test comparable with MyChoice such as shallowHRDv2 might thus also help estimating the benefit of prescribing niraparib in BRCAwt AOC patients in first-line. Similarly, and more globally, HRD testing might be useful for other tumor types besides ovarian carcinomas (Coussy, F. & Bidard, F. C. Expanding biomarkers for PARP inhibitors. Nat Cancer 3, 1141-1143 (2022); Gruber, J. J., et al. A phase II study of talazoparib monotherapy in patients with wild-type BRCA1 and BRCA2 with a mutation in other homologous recombination genes. Nat Cancer 3, 1181-1191 (2022)) and our test was developed in pan-cancer samples.

Other teams also participated in the EHEI and new tests to detect HRD have been validated on tumor samples from the PAOLA-1 trial (Loverix, L., et al. Predictive value of the Leuven HRD test compared with Myriad myChoice PLUS on 468 ovarian cancer samples from the PAOLA-1/ENGOT-ov25 trial (LBA 6). Gynecologic Oncology 166, S51-S52 (2022); Willing, E.-M., et al. 2022-RA-873-ESGO Validation study of the 'NOGGO-GIS ASSAY' based on ovarian cancer samples from the first-line PAOLA-1/ENGOT-ov25 phase-III trial. International Journal of Gynecologic Cancer 32, A370-A370 (2022); Buisson, A., et al. 2022-RA-913-ESGO Clinical performance evaluation of a novel deep learning solution for homologous recombination deficiency detection. International Journal of Gynecologic Cancer 32, A277-A278 (2022); Leman, R., et al. 2022-RA-935-ESGO Development of an academic genomic instability score for ovarian cancers. International Journal of Gynecologic Cancer 32, A280-A280 (2022); Christinat, Y., et al. 2022-RA-567-ESGO The Geneva HRD test: clinical validation on 469 samples from the PAOLA-1 trial. International Journal of Gynecologic Cancer 32, A238-A239 (2022). All of them reported a decrease in non-contributory results compared with MyChoice and overall satisfactory clinical performances in predicting PFS benefit from ola+bev combination. However, the majority of these tests are based on NGS capture panels coupled with sequencing of homologous recombination repair genes. Thus, laboratories wishing to implement these tests would be forced to change their already validated method of detecting homologous recombination repair gene alterations, whereas this is not requested using shallowHRDv2 based on pre-capture library sequencing. Detection of HRD status using single nucleotide polymorphism arrays remains independent of homologous recombination repair genes sequencing but has the disadvantage of being more expensive and time consuming than sWGS.

In conclusion, the shallowHRDv2 test is robust, cost-effective, easy to implement, clinically validated and can be considered as a reference test to detect HRD, along with the MyChoice test. Moreover, as a large dataset of different tumor types was used to develop the shallowHRD pipelines, we are confident that the shallowHRDv2 test will be applicable to predict response to PARPi in future clinical trials.

## Claims

1. A method for diagnosing a homologous recombination deficiency (HRD) in a tumor, said method comprising the steps of:
- evaluating the number of large-scale genomic alterations (LGA number) by obtaining the copy number alterations (CNA) profile by shallow coverage whole genome sequencing (sWGS) in a tumor sample,
- determining an LGA-score which corresponds to said LGA number adjusted by the genome complexity of the tumor and the presence of a marker selected from the group of markers consisting of (1) cyclin dependent kinase 12 (CDK12) mutation-associated phenotype with multiple interstitial gains in the CNA profile, (2) Cyclin E1 (CCNE1) amplification, (3) human epidermal growth factor receptor-2 (HER2) amplification and (4) a multifocal amplification phenotype corresponding to more than two chromosome arms carrying at least one high copy gain of any given region,
wherein a high LGA-score is associated with HRD and a low LGA-score is associated with HR pathway proficiency (HRP),
wherein genome complexity is determined by assigning the sample as being "simple" or "complex", wherein a genome that has two most abundant copy number levels accounting for more than 70% of the genome is assigned as "simple", and otherwise assigned as "complex", wherein a "simple" genome adjusts the diagnosis towards HRD, and a "complex" genome adjusts the diagnosis towards HRP,
wherein the presence of said marker adjusts the diagnosis towards HRP.

2. The method according to claim 1, wherein said sample is selected from the group consisting of fresh tumor samples obtained from the subject and preserved tumor samples such as frozen tumor samples and formalin-fixed paraffin embedded (FFPE) tumor samples.

3. The method according to claim 2, wherein said tumor sample is a formalin-fixed paraffin embedded (FFPE) sample of the tumor and wherein the sWGS CNA profile has been corrected by eliminating the FFPE noise profile.

4. The method according to any one of claims 1 to 3, wherein borderline cases regarding the classification of HRD are resolved by taking into account:
- the genome complexity of the tumor;
- LGA_max, the upper bound of LGA number found in segmented copy number profile;
- the presence of a marker selected from the group of markers consisting of (1) cyclin dependent kinase 12 (CDK12) mutation-associated phenotype with multiple interstitial gains in the CNA profile, (2) Cyclin E1 (CCNE1) amplification, (3) human epidermal growth factor receptor-2 (HER2) amplification and (4) multifocal amplification phenotype.

5. The method according to any one of claims 1 to 4 wherein said tumor is a breast tumor.

6. The method according to any one of claims 1 to 4, wherein said tumor is an ovarian tumor.

7. A method for predicting the efficacy of a treatment comprising a PARP inhibitor (PARPi) and/or an alkylating agent in a patient suffering from cancer, wherein said method comprises diagnosing HRD in a tumor sample according to the method of any one of claims 1 to 6.

8. A PARPi and/or an alkylating agent for use in a method for treating a cancer in a patient, wherein said patient has been diagnosed as having a tumor presenting a HRD according to the method of any one of claims 1 to 6.

## Patentansprüche

1. Verfahren zur Diagnose einer homologen Rekombinationsdefizienz (HRD) in einem Tumor, wobei das Verfahren die folgenden Schritte umfasst:
• Bestimmen der Anzahl großskaliger genomischer Alterationen (LGA-Anzahl) durch Erhalten des Profils von Kopienanzahlalterationen (CNA) mittels Ganzgenomsequenzierung mit geringer Abdeckung (sWGS) in einer Tumorprobe,
• Bestimmen von:
• einem LGA-Score, welcher der LGA-Anzahl entspricht, bereinigt um die Genomkomplexität des Tumors und das Vorhandensein eines Markers, der ausgewählt ist aus der Gruppe von Markern bestehend aus (1) einem mit einer Mutation der Cyclin-abhängigen Kinase 12 (CDK12) assoziierten Phänotyp mit multiplen interstitiellen Gewinnen im CNA-Profil, (2) einer Cyclin E1 (CCNE1)-Amplifikation, (3) einer humanen epidermalen Wachstumsfaktorrezeptor-2 (HER2)-Amplifikation und (4) einem multifokalen Amplifikationsphänotyp, der mehr als zwei Chromosomenarmen entspricht, die mindestens einen hohen Kopienanzahlgewinn einer beliebigen gegebenen Region tragen,<p>wobei ein hoher LGA-Score mit HRD assoziiert ist und ein niedriger LGA-Score mit HR-Signalweg-Profizienz (HRP) assoziiert ist,</p><p>wobei die Genomkomplexität bestimmt wird, indem die Probe als "einfach" oder "komplex" eingestuft wird, wobei ein Genom, das zwei am häufigsten vorkommende Kopienanzahlniveaus aufweist, die für mehr als 70 % des Genoms verantwortlich sind, als "einfach" eingestuft wird, und andernfalls als "komplex" eingestuft wird, wobei ein "einfaches" Genom die Diagnose in Richtung HRD anpasst, und ein "komplexes" Genom die Diagnose in Richtung HRP anpasst,</p><p>wobei das Vorhandensein des Markers die Diagnose in Richtung HRP anpasst.</p>

2. Verfahren nach Anspruch 1, wobei die Probe ausgewählt ist aus der Gruppe bestehend aus frischen Tumorproben, die von dem Subjekt erhalten wurden, und konservierten Tumorproben, wie gefrorenen Tumorproben und Formalin-fixierten, Paraffin-eingebetteten (FFPE) Tumorproben.

3. Verfahren nach Anspruch 2, wobei die Tumorprobe eine Formalinfixierte, Paraffin-eingebettete (FFPE) Probe des Tumors ist und wobei das sWGS-CNA-Profil durch Eliminieren des FFPE-Rauschprofils korrigiert wurde.

4. Verfahren nach einem der Ansprüche 1 bis 3, wobei Grenzfälle bezüglich der Klassifizierung von HRD gelöst werden unter Berücksichtigung:
• der Genomkomplexität des Tumors;
• von LGAₘₐₓ, der Obergrenze der in einem segmentierten Kopienanzahlprofil gefundenen LGA-Anzahl;
• des Vorhandenseins eines Markers, der ausgewählt ist aus der Gruppe von Markern bestehend aus (1) einem mit einer Mutation der Cyclin-abhängigen Kinase 12 (CDK12) assoziierten Phänotyp mit multiplen interstitiellen Gewinnen im CNA-Profil, (2) einer Cyclin E1 (CCNE1)-Amplifikation, (3) einer humanen epidermalen Wachstumsfaktorrezeptor-2 (HER2)-Amplifikation und (4) einem multifokalen Amplifikationsphänotyp.

5. Verfahren nach einem der Ansprüche 1 bis 4, wobei der Tumor ein Brusttumor ist.

6. Verfahren nach einem der Ansprüche 1 bis 4, wobei der Tumor ein Ovarialtumor ist.

7. Verfahren zur Vorhersage der Wirksamkeit einer Behandlung umfassend einen PARP-Inhibitor (PARPi) und/oder ein Alkylierungsmittel bei einem an Krebs leidenden Patienten, wobei das Verfahren das Diagnostizieren von HRD in einer Tumorprobe gemäß dem Verfahren nach einem der Ansprüche 1 bis 6 umfasst.

8. PARPi und/oder ein Alkylierungsmittel zur Verwendung in einem Verfahren zur Behandlung eines Krebses bei einem Patienten, wobei bei dem Patienten ein Tumor diagnostiziert wurde, der eine HRD aufweist, gemäß dem Verfahren nach

## Revendications

1. Procédé de diagnostic d'une déficience de la recombinaison homologue (HRD) dans une tumeur, ledit procédé comprenant les étapes de :
• l'évaluation du nombre d'altérations génomiques à grande échelle (nombre LGA) par l'obtention du profil d'altérations du nombre de copies (CNA) par séquençage du génome entier à faible couverture (sWGS) dans un échantillon de tumeur,
• la détermination :
• d'un score LGA qui correspond audit nombre LGA ajusté par la complexité du génome de la tumeur et la présence d'un marqueur choisi dans le groupe de marqueurs constitué par (1) un phénotype associé à une mutation de la kinase dépendante des cyclines 12 (CDK12) avec des gains interstitiels multiples dans le profil CNA, (2) une amplification de la cycline E1 (CCNE1), (3) une amplification du récepteur 2 du facteur de croissance épidermique humain (HER2) et (4) un phénotype d'amplification multifocale correspondant à plus de deux bras chromosomiques portant au moins un gain de nombre de copies élevé de n'importe quelle région donnée,<p>dans lequel un score LGA élevé est associé à la HRD et un score LGA faible est associé à la compétence de la voie HR (HRP),</p><p>dans lequel la complexité du génome est déterminée en qualifiant l'échantillon de « simple » ou « complexe », dans lequel un génome qui présente deux niveaux de nombre de copies les plus abondants représentant plus de 70 % du génome est qualifié de « simple », et autrement qualifié de « complexe », dans lequel un génome « simple » ajuste le diagnostic vers la HRD, et un génome « complexe » ajuste le diagnostic vers la HRP,</p><p>dans lequel la présence dudit marqueur ajuste le diagnostic vers la HRP.</p>

2. Procédé selon la revendication 1, dans lequel ledit échantillon est choisi dans le groupe constitué par les échantillons de tumeur frais obtenus chez le sujet et les échantillons de tumeur conservés tels que les échantillons de tumeur congelés et les échantillons de tumeur fixés au formol et inclus en paraffine (FFPE).

3. Procédé selon la revendication 2, dans lequel ledit échantillon de tumeur est un échantillon de la tumeur fixé au formol et inclus en paraffine (FFPE) et dans lequel le profil CNA sWGS a été corrigé en éliminant le profil de bruit FFPE.

4. Procédé selon l'une quelconque des revendications 1 à 3, dans lequel les cas limites concernant la classification de la HRD sont résolus en prenant en compte :
• la complexité du génome de la tumeur ;
• LGAₘₐₓ, la limite supérieure du nombre LGA trouvé dans un profil de nombre de copies segmenté ;
• la présence d'un marqueur choisi dans le groupe de marqueurs constitué par (1) un phénotype associé à une mutation de la kinase dépendante des cyclines 12 (CDK12) avec des gains interstitiels multiples dans le profil CNA, (2) une amplification de la cycline E1 (CCNE1), (3) une amplification du récepteur 2 du facteur de croissance épidermique humain (HER2) et (4) un phénotype d'amplification multifocale.

5. Procédé selon l'une quelconque des revendications 1 à 4, dans lequel ladite tumeur est une tumeur du sein.

6. Procédé selon l'une quelconque des revendications 1 à 4, dans lequel ladite tumeur est une tumeur de l'ovaire.

7. Procédé de prédiction de l'efficacité d'un traitement comprenant un inhibiteur de PARP (PARPi) et/ou un agent alkylant chez un patient souffrant d'un cancer, dans lequel ledit procédé comprend le diagnostic de la HRD dans un échantillon de tumeur selon le procédé de l'une quelconque des revendications 1 à 6.

8. PARPi et/ou agent alkylant pour une utilisation dans une méthode de traitement d'un cancer chez un patient, dans lequel ledit patient a été diagnostiqué comme ayant une tumeur présentant une HRD selon le procédé de l'une quelconque des revendications 1 à 6.
